# EUROPEAN PATENT APPLICATION

(11) **EP 4 431 036 A1**
(43) Date of publication of application: **18.09.2024**
(21) Application number: 22891843.9
(22) Date of filing: 01.11.2022
(51) Int. Cl.: A61B 18/02

(54) **CRYOABLATION NEEDLE HAVING J-T SLOT SLEEVE**

(30) Priority: 11.11.2021 CN 202111329723
(71) Applicant: Shanghai Chest Hospital, Shanghai 200030 (CN); Accu Target Medipharma (Shanghai) Co., Ltd., Shanghai 201318 (CN)
(72) Inventor: SUN, Jiayuan, Shanghai 200030 (CN); YANG, Chi, Shanghai 201318 (CN); XU, Binkai, Shanghai 201318 (CN); XIE, Fangfang, Shanghai 200030 (CN); GU, Chuanjia, Shanghai 200030 (CN); FENG, Xintong, Shanghai 200030 (CN)
(74) Representative: EP&C
(86) International application number: PCT/CN2022/128869
(87) International publication number: WO 2023/083047

(57) **Abstract**

The present invention discloses a cryoablation needle having a J-T slot sleeve, including: a vacuum wall, a J-T slot and a J-T slot sleeve, where the vacuum wall includes: a needle rod and an inner tube; the needle rod is provided with a needle tip at a distal end; the inner tube penetrates through the needle rod, and an cavity is formed between the inner tube and the needle rod; a first preset distance exists between a distal end of the inner tube and the distal end of the needle rod; the J-T slot and the J-T slot sleeve are sleeved inside the inner tube; the J-T slot sleeve is sleeved outside a distal end of the J-T slot; the J-T slot sleeve can move relative to the J-T slot; in areas in which the vacuum wall is distributed in the axis direction of the vacuum wall, an area in which the cavity is located is a vacuum insulation area, and an area in which the first preset distance exists is a target area; and a distal end of the J-T slot sleeve at least has two adjusting positions relative to the vacuum wall, a first adjusting position being located in the target area, and a second adjusting position being located in the vacuum insulation area. According to the present invention, the distal end of the J-T slot sleeve is switched between the at least two adjusting positions, such that a cooling rate is greatly increased.

## Description

### TECHNICAL FIELD

The present invention relates to the technical field of cryoablation, and in particular to a cryoablation needle having a J-T slot sleeve.

### BACKGROUND

Cryoablation is a treatment approach that uses low temperature to destroy diseased tissues, which is considered to be an efficient and minimally invasive method to treat malignant tumors. The cryoablation is easy to operate, has few complications, and can effectively relieve pain. At the same time, ice balls formed by ablation have clear boundaries and are easy to observe, and lesions near large blood vessels or important organs can be safely ablated. The cryoablation may also adopt a multi-needle freezing manner, so that a wider ablation range is achieved, which is suitable for large lesions and irregular lesions.

In the process of cell freezing, ice crystals are firstly formed outside cells, which causes the concentration of extracellular solute to increase, resulting in a hypertonic environment, and water in the cells enters the outside of the cells, resulting in intracellular dehydration. The cells that lose water become shrunk and cell membranes are deformed, resulting in a "solution damage" in a high-concentration toxic environment. At the same time, ice crystals formed in the cells directly damage organelles and the cell membranes, causing further necrosis, commonly known as an "intracellular ice damage". The intracellular ice damage directly damages cell structures, and therefore is more destructive to the cells. Generally, the lower a cooling rate of the cells, the greater the probability of the "solution damage", and the higher the cooling rate, the easier it is to induce the "intracellular ice damage". Therefore, the higher cooling rate is generally pursued in the process of tumor cryoablation, which can kill tumors more thoroughly and greatly save surgery time.

The development of the cryoablation has undergone three stages. The first stage is a liquid nitrogen conveying and refrigeration technology, which conveys liquid nitrogen at -196°C to a needle tip of a cryoablation needle by a low driving pressure to achieve the purpose of cryoablation. Since a cold source of this technology completely relies on the liquid nitrogen, which is located in a main machine or liquid nitrogen barrel and has a long conveying distance from the needle tip, during the liquid nitrogen conveying process, only when a whole conveying pipeline reaches -196°C, the temperature of the needle tip can reach -196°C. Therefore, the cooling rate of liquid nitrogen refrigeration is the lowest in the prior art. The second stage is a direct throttling refrigeration technology, which uses the principle of "Joule Thompson Effect" (J-T for short), and conveys ultra-high pressure gas at room temperature to a J-T slot (a capillary tube that produces J-T effect) inside the cryoablation needle to directly throttle to produce low temperature. The cooling rate of this technology is relatively the highest in the prior art. However, structures such as the J-T slot and a finned tube inside the needle tip may consume a part of cold, thus prolonging cooling time. In addition, the ultra-high pressure gas used is not highly popularized and is expensive, resulting in difficulty in the promotion of this technology. The third stage is a pre-cooled throttling refrigeration technology, the principle of which is to pre-cool normal industrial gas that is at room temperature by a supporting main machine, and then convey pre-cooled normal industrial gas to the J-T slot inside the cryoablation needle to produce ablation temperature that is lower than preset temperature by throttling. This technology solves the problem that gas sources are expensive and scarce. Furthermore, this technology combines a throttling refrigeration technology, and thus the cooling rate thereof is obviously higher than that of the liquid nitrogen refrigeration technology, but is still lower than that of the direct throttling refrigeration technology.

### SUMMARY

For problems existing in the prior art, the present invention provides a cryoablation needle having a J-T slot sleeve, to solve the problem of low cooling rate in the prior art.

In order to resolve the above technical problem, the present invention is implemented through the following technical solutions:

The present invention provides a cryoablation needle having a J-T slot sleeve, including: a vacuum wall, a J-T slot and a J-T slot sleeve, where
the vacuum wall includes: a needle rod and an inner tube;
the needle rod is provided with a needle tip at a distal end;
the inner tube penetrates through the needle rod, and a cavity is formed between the inner tube and the needle rod, the cavity being capable of forming a vacuum;
in an axis direction of the vacuum wall, a first preset distance exists between a distal end of the inner tube and the distal end of the needle rod; the distal end of the inner tube is an end of the inner tube close to the needle tip;
the J-T slot sleeve is sleeved outside a distal end of the J-T slot or sleeved inside the distal end of the J-T slot; the distal end of the J-T slot is an end of the J-T slot close to the needle tip;
the J-T slot and the J-T slot sleeve penetrate through the inner tube;
in areas in which the vacuum wall is distributed in the axis direction of the vacuum wall, an area in which the cavity is located is a vacuum insulation area, and an area in which the first preset distance exists is a target area;
the distal end of the J-T slot is located in the vacuum insulation area;
the J-T slot is capable of moving in the axis direction of the vacuum wall relative to the J-T slot, and a dynamic seal is formed between the J-T slot sleeve and the J-T slot;
a distal end of the J-T slot sleeve is capable of being switched between at least two adjusting positions relative to the vacuum wall, the at least two adjusting positions including: a first adjusting position and a second adjusting position;
the first adjusting position is located in the target area;
the second adjusting position is located in the vacuum insulation area;
when the distal end of the J-T slot sleeve is located at the first adjusting position, in the axis direction of the vacuum wall, a second preset distance exists between the distal end of the J-T slot sleeve and the needle tip, and the second preset distance at least ensures that an ice ball formed by freezing is wrapped around the needle tip; and
when the distal end of the J-T slot sleeve is located at the second adjusting position, in the axis direction of the vacuum wall, a third preset distance exists between the distal end of the J-T slot sleeve and a distal end of the vacuum insulation area, and the third preset distance at least ensures that a refrigerant directly returns from the inside of the vacuum insulation area after being sprayed from the J-T slot sleeve, the distal end of the vacuum insulation area being an end of the vacuum insulation area close to the needle tip.

Preferably, the cryoablation needle having the J-T slot sleeve further includes: a J-T slot sleeve adjusting apparatus, where the J-T slot sleeve adjusting apparatus is configured to enable the distal end of the J-T slot sleeve to be switched between the at least two adjusting positions.

Preferably, the J-T slot sleeve adjusting apparatus includes: a push tube and a mandrel, where
the mandrel is arranged in the axis direction of the vacuum wall;
the push tube penetrates through the mandrel;
a distal end of the push tube is connected to a proximal end of the J-T slot sleeve; the distal end of the push tube is an end of the push tube close to the needle tip; and
the push tube and the J-T slot sleeve are capable of being controlled to synchronously move in the axis direction, to switch the distal end of the J-T slot sleeve between the adjusting positions.

Preferably, the cryoablation needle having the J-T slot sleeve further includes: a sealing assembly, where the sealing assembly is configured to form a dynamic seal between the mandrel and the push tube.

Preferably, the sealing assembly includes: a sealing ring, a sealing slot and a sealing press piece, where
the sealing slot is fixedly sealed with a proximal end of the mandrel;
the sealing ring is arranged between the mandrel and the sealing slot, and the sealing press piece is arranged between the sealing ring and the sealing slot; and
the sealing press piece is capable of being controlled to extrude axially, so that the sealing ring radially extrudes the mandrel, to form the dynamic seal between the mandrel and the push tube.

Preferably, the cryoablation needle having the J-T slot sleeve further includes: a spring and a clamping piece, where
one end of the spring is capable of synchronously with the distal end of the J-T slot sleeve, and is further connected to the clamping piece; the clamping piece is capable of entering and exiting from a clamped position;
the other end of the spring is fixed relative to the vacuum wall;
when the clamping piece is located at the clamped position, the spring is limited by the clamping piece to keep in a deformation state, and the distal end of the J-T slot sleeve is located at the second adjusting position;
the deformation state is a compression state or a tension state; and
when the clamping piece exits from the clamped position, the spring is capable of generating a restoring force for restoring from the deformation state to a natural state, and the restoring force is capable of driving the distal end of the J-T slot sleeve to enter the first adjusting position from the second adjusting position.

Preferably, the clamping piece includes: a positioning pin and a C-shaped ring, where
the positioning pin is arranged on the C-shaped ring;
the C-shaped ring is wrapped around an outer wall with a fixed position relative to the vacuum wall; and
when the distal end of the J-T slot sleeve is located at the first adjusting position, the positioning pin is configured to keep the spring in the deformation state.

Preferably, the cryoablation needle having the J-T slot sleeve further includes: a sliding block and a handle, where
the handle is arranged at a proximal end of the vacuum wall, and a position of the handle is fixed relative to the vacuum wall; the proximal end of the vacuum wall is an end of the vacuum wall far away from the needle tip;
the sliding block is connected to a proximal end of the push tube, and the sliding block is also directly or indirectly connected to the clamping piece; the proximal end of the push tube is an end of the push tube far away from the needle tip;
the sliding block, the push tube and the J-T slot sleeve are capable of being controlled to synchronously move in the axis direction, to switch the distal end of the J-T slot sleeve between the adjusting positions;
the handle is provided with a handle positioning slot and the sliding block is provided with a sliding block positioning slot; and
the positioning pin is capable of being controlled to be synchronously inserted into the sliding block positioning slot and the handle positioning slot, so that positions of the sliding block, the handle and the vacuum wall are relatively fixed, and in this case, the distal end of the J-T slot sleeve is located at the second adjusting position.

Preferably, the vacuum wall further includes: an outer tube, where
a distal end of the outer tube is hermetically connected to a proximal end of the needle rod, a proximal end of the outer tube is hermetically connected to a proximal end of the inner tube; the distal end of the outer tube is an end of the outer tube close to the needle tip, and the proximal end of the outer tube is an end of the outer tube far away from the needle tip;
an outer diameter of the outer tube is greater than an outer diameter of the needle rod, and an inner diameter of the outer tube is greater than an inner diameter of the needle rod;
from the distal end to the proximal end of the inner tube, the inner tube sequentially comprises: an inner tube front section and an inner tube rear section; an outer diameter of the inner tube rear section is greater than an outer diameter of the inner tube front section; an inner diameter of the inner tube rear section is greater than an inner diameter of the inner tube front section; and
the inner tube front section penetrates through the needle rod; and the inner tube rear section penetrates through the outer tube.

Preferably, a dynamic sealing point between the J-T slot sleeve and the J-T slot is located at a proximal end of the J-T slot sleeve; and
the dynamic sealing point is located inside the inner tube rear section.

Preferably, the cryoablation needle having the J-T slot sleeve further includes: a temperature measuring wire, where
a distal end of the temperature measuring wire is a temperature measuring point; the distal end of the temperature measuring wire is an end of the temperature measuring wire close to the needle tip; and
the temperature measuring point is arranged at the distal end of the J-T slot sleeve and used for measuring temperature at the distal end of the J-T slot sleeve.

Compared with the prior art, the present invention has the following advantages:
(1) According to the cryoablation needle having the J-T slot sleeve provided in the present invention, the J-T slot sleeve is sleeved on the J-T slot, so that the J-T slot sleeve is capable of moving axially relative to the J-T slot. When the distal end of the J-T slot sleeve is located inside the vacuum insulation area, a refrigerant fluid is introduced into the J-T slot to start freezing. All conveying pipelines at a main machine side and a cryoablation needle side can be pre-purged (cooled), and no cold is consumed at a target area during a pre-purging process. Therefore, all cold is used for cooling the conveying pipelines, and thus the cooling rate of the conveying pipeline process is the highest. Furthermore, no cold is released at the target area during the pre-purging process, so that there are no frosting and freezing phenomena in the target area, and then formal surgery can be performed directly.
(2) According to the cryoablation needle having the J-T slot sleeve provided in the present invention, only the target area of the vacuum wall of the pre-purged cryoablation needle is not cooled. When the distal end of the J-T slot sleeve is located at the target area, the refrigerant fluid is introduced into the J-T slot to start freezing, and all heat loads only come from the target area and tumor tissues outside the target area. Therefore, the cooling rate of this freezing process can be greatly increased.
(3) According to the cryoablation needle having the J-T slot sleeve provided in the present invention, after a needling test procedure ends, a pre-purging mode can be kept enabled, the inside (the distal end of the J-T slot sleeve) of the vacuum insulation area is kept at the lowest temperature. Since the target area does not release any cold, operations such as puncturing, scanning and positioning can be performed, and then the cryoablation needle is adjusted to a freezing mode after the puncturing is in place. In this case, the inside of the target area is directly reduced from the normal temperature to the lowest temperature instantly. Therefore, ultimate rapid cooling of the formal surgery can be achieved.
(4) The cryoablation needle having the J-T slot sleeve provided in the present invention is wide in application range, and can be applied to all existing cryoablation technologies: liquid nitrogen conveying and refrigeration technology, direct throttling refrigeration technology and pre-cooled throttling refrigeration technology. The cryoablation needle having the J-T slot sleeve is not only applicable to percutaneous puncture cryoablation instruments, but also applicable to cryoablation instruments of natural orifice transluminal surgery.

Certainly, implementing any product of the present invention does not necessarily need to simultaneously achieve all the advantages described above.

### BRIEF DESCRIPTION OF DRAWINGS

In order to explain the technical solutions in the embodiments of the present invention or in the prior art more clearly, the drawings used in the description of the embodiments or the prior art will be briefly introduced below. Obviously, the drawings in the following description are merely some embodiments of the present invention. For those of ordinary skill in the art, other drawings can be obtained based on these drawings without creative efforts.
FIG. 1 is a diagram of a J-T slot adjustment principle of a hard cryoablation needle having a J-T slot sleeve according to an embodiment of the present invention;
FIG. 2 is a diagram of a J-T slot adjustment principle of a flexible cryoablation needle having a J-T slot sleeve according to an embodiment of the present invention;
FIG. 3 is a diagram of a pre-purging mode of a hard cryoablation needle having a J-T slot sleeve according to a preferred embodiment of the present invention;
FIG. 4 is an enlarged view of a portion I in FIG. 3;
FIG. 5 is a diagram of a freezing mode of a hard cryoablation needle having a J-T slot sleeve according to a preferred embodiment of the present invention;
FIG. 6 is an enlarged view of a portion II in FIG. 5;
FIG. 7 is a schematic diagram of a vacuum wall of a flexible cryoablation needle having a J-T slot sleeve according to a preferred embodiment of the present invention;
FIG. 8 is a diagram of a pre-purging mode of a flexible cryoablation needle having a J-T slot sleeve according to a preferred embodiment of the present invention;
FIG. 9 is a diagram of a freezing mode of a flexible cryoablation needle having a J-T slot sleeve according to a preferred embodiment of the present invention;
FIG. 10 is a diagram of a core adjustment mechanism of a flexible cryoablation needle having a J-T slot sleeve according to a preferred embodiment of the present invention;
FIG. 11 is a schematic diagram of a sliding block according to a preferred embodiment of the present invention; and
FIG. 12 is a schematic diagram of a clamping ring according to a preferred embodiment of the present invention.

### Description of reference signs: 1 - J-T slot,

2 - vacuum wall,
21 - needle rod,
211 - needle tip,
22 - inner tube,
221 - inner tube front section,
222 - inner tube rear section,
23 - outer tube,
24 - gasket,
25 - target area,
26 - vacuum insulation area,
27 - spring stop,
28 - vacuum tee,
281 - tee connecting portion,
282 - tee bypass,
291 - vacuum connecting tube,
292 - vacuum hose,
293 - return gas connecting tube,
294 - shunt,
3 - mandrel,
4 - finned tube,
5 - sealing assembly,
51 - sealing ring,
52 - sealing slot,
53 - sealing press piece,
6 - gas intake tube,
7 - gas return tube,
8 - sliding block,
81 - guiding tube,
82 - sliding block positioning slot
83 - middle fixing hole,
84 - gas intake/return tube guiding hole,
9 - handle,
91 - handle positioning slot,
10 - clamping piece,
101 - hand-held portion,
102 - positioning pin,
103 - C-shaped ring,
120 - spring,
13 - outer sleeve,
14 - temperature measuring wire,
141 - temperature measuring point,
17 - push tube,
173 - temperature measuring notch,
174 - gas intake notch,
175 - push tube connecting section,
18 - J-T slot sleeve,
181 - sleeve main section,
182 - sleeve sealing section,
183 - sleeve connecting section,
19 - sealing gasket.

### DESCRIPTION OF EMBODIMENTS

The technical solutions in embodiments of the present invention will be clearly and fully described in combination with the drawings of the embodiments of the present invention; it is obvious that the described embodiments are only a part of, and not all embodiments of, present invention. Based on the embodiments of the present invention, all other embodiments obtained by those of ordinary skill in the art without creative efforts should fall within the protection scope of the present invention.

In the description of the specification of the present invention, it should be understood that the term "upper portion", "lower portion", "upper end", "lower end", "upper surface", "lower surface" or the like indicates an orientation or positional relationship based on that shown in the drawings, which is merely for ease of description and simplicity of description, and is not intended to indicate or imply that the apparatus or element referred to must have a particular orientation, be constructed and operate in a particular orientation, and therefore cannot be construed as a limitation on the present invention.

In the description of the specification of the present invention, the terms "first" and "second" are used for descriptive purposes only, and cannot be construed as indicating or implying relative importance or implicitly indicating the number of technical features indicated. Therefore, a feature defined with "first" and "second" may explicitly or implicitly include one or more of the features.

In the description of the present invention, "a plurality of" means multiple, such as two, three, four or the like, unless specifically defined otherwise.

The technical solutions of the present invention will be described in detail below by specific embodiments. The following several specific embodiments may be mutually combined, and same or similar concepts or processes may not be repeatedly described in some embodiments.

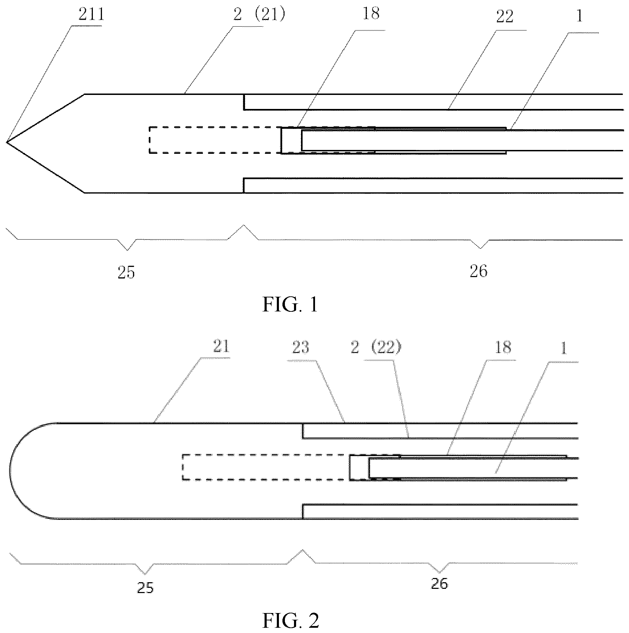

As shown in FIG. 1 and FIG. 2, which each is a diagram of a J-T slot adjustment principle of a cryoablation needle having a J-T slot sleeve according to an embodiment of the present invention.

Referring to FIG. 1 and FIG. 2, the cryoablation needle having the J-T slot sleeve according to this embodiment includes: a vacuum wall 2, a J-T slot 1 and a J-T slot sleeve 18.

The vacuum wall 2 includes: a needle rod 21 and an inner tube 22, and the needle rod 21 is provided with a needle tip 211 at a distal end. The inner tube 22 penetrates through the needle rod 21, and a cavity is formed between the inner tube 22 and the needle rod 21, the cavity being a cavity that can form a vacuum, which may be a permanent vacuum cavity or a real-time vacuum cavity. The vacuum cavity plays a role in heat insulation and preventing frostbite of normal tissues.

A first preset distance (the first preset distance can be understood as a spacing distance in the axis direction of the vacuum wall) exists between a distal end of the inner tube 22 and the distal end of the needle rod. The distal end of the inner tube 22 is an end of inner tube 22 close to the needle tip 211.

In areas in which the vacuum wall is distributed in the axis direction of the vacuum wall, since the vacuum cavity plays a role in heat insulation, an area in which the cavity is located is a vacuum insulation area 26, and an area in which the first preset distance exists is a target area 25.

The J-T slot sleeve 18 is sleeved outside a distal end of the J-T slot 1 or sleeved inside the distal end of the J-T slot 1; and the distal end of the J-T slot 1 is an end of the J-T slot 1 close to the needle tip 211. The J-T slot 1 and J-T slot sleeve 18 penetrate through the inner tube 22.

In FIG. 1 and FIG. 2, the J-T slot sleeve 18 is sleeved outside the distal end of the J-T slot 1. In a different embodiment, the J-T slot sleeve 18 may alternatively be sleeved inside the distal end of the J-T slot 1.

The J-T slot sleeve 18 can move in the axis direction of the vacuum wall relative to the J-T slot 1, and a dynamic seal is formed between the J-T slot sleeve 18 and the J-T slot 1.

A distal end of the J-T slot sleeve can be switched between at least two adjusting positions relative to the vacuum wall (for example, switching can be realized by moving in the axis direction of the vacuum wall), the at least two adjusting positions including: a first adjusting position and a second adjusting position. When the distal end of the J-T slot sleeve 18 is located at the first adjusting position, the distal end of the J-T slot sleeve 18 is located in the target area 25, which can be understood as being in a freezing mode, as shown by dashed lines in FIG. 1 and FIG. 2; and the distal end of the J-T slot 1 is an end of the J-T slot 1 close to the needle tip 211. When the distal end of the J-T slot sleeve 18 is located at the second adjusting position, the distal end of the J-T slot sleeve 18 is located in the vacuum insulation area 26, which can be understood as being in a pre-purging mode, as shown by solid lines in FIG. 1 and FIG. 2.

When the distal end of the J-T slot sleeve 18 is located at the first adjusting position, in the axis direction of the vacuum wall, a second preset distance exists between the distal end of the J-T slot sleeve 18 and the needle tip, and the second preset distance at least ensures that an ice ball formed by freezing is wrapped around the needle tip. That is, a refrigerant fluid returns from the inside of the target area and the inside of the vacuum insulation area after being sprayed from the J-T slot sleeve, where the refrigerant fluid exchanges heat with substances outside the whole target area during the process of returning from the inside of the target area.

When the distal end of the J-T slot sleeve 18 is located at the second adjusting position, in the axis direction of the vacuum wall, a third preset distance exists between the distal end of the J-T slot sleeve 18 and a distal end of the vacuum insulation area, and the third preset distance at least ensures that the refrigerant fluid directly returns from the inside of the vacuum insulation area after being sprayed from the J-T slot sleeve. Only a relatively static refrigerant exists in the target area, which will not exchange heat with the substances outside the target area. That is, the refrigerant does not release any cold in the target area during a freezing process. The distal end of the vacuum insulation area is an end of the vacuum insulation area close to the needle tip.

The first preset distance, the second preset distance and the third preset distance may be understood as spacing distances in the axis direction of the vacuum wall 2. In addition, an axial direction mentioned later can be understood as the axis direction of the vacuum wall 2.

In an embodiment, the vacuum wall is a hard-material vacuum wall, which can be applied to percutaneous cryoablation instruments. As shown in FIG. 1, the needle tip 211 is in the form of a pinpoint.

In an embodiment, the vacuum wall is a flexible-material vacuum wall, which can be applied to natural orifice transluminal ablation instruments. As shown in FIG. 2, the needle tip 211 is in the form of a circular arc. Preferably, the needle rod 21 and the inner tube 22 may be made of flexible nonmetallic materials or freely bendable metallic materials, such as PTFE or PTFE braided tubes or stainless-steel bellows.

In an embodiment, a use process of the cryoablation needle having the J-T slot sleeve is as follows: Before surgery, the cryoablation needle in the pre-purging mode is taken out to be mutually connected to a main machine. The needle rod 21 (at least the target area 25) of the cryoablation needle is inserted into physiological saline to start a needling test function. A rewarming operation is firstly performed during needling test. When the temperature of the needle tip rises to a certain temperature value within a certain time, it proves that a rewarming function is normal. Then, a program automatically performs a freezing operation. When the temperature of the needle tip is reduced to a certain temperature value within a certain time, it proves that a freezing function is normal. In this case, the time the needle tip is kept at the lowest temperature can be properly prolonged for sufficient pre-purging, and then the needling test is automatically stopped. During the freezing operation, whether there is a frosting phenomenon in the vacuum insulation area 26 is observed. If there is no frosting phenomenon, it proves that a heat insulation function is normal. Whether there is air leakage in the needle tip immersed in the physiological saline is observed during the whole process. If there is not air leakage, it proves that gas tightness is normal. After the needling test, conveying pipelines of both the main machine and the cryoablation needle have been pre-purged (cooled). Then, the freezing function can be enabled (or a separately configured pre-purging function can be enabled) at first, and freezing at this stage can be carried out at a lower working pressure, or gas can be intermittently introduced, so that the temperature at the distal end of the J-T slot can be kept at the lowest temperature while gas consumption can be reduced. Next, under the condition of keeping the freezing function enabled, percutaneous puncturing can be performed under the guidance of imaging, so that the needle tip can reach an expected tumor position. In this case, the J-T slot can be adjusted to move toward the distal end, and stop at the first adjusting position, to switch to the freezing mode. Since the whole conveying pipeline is already in a low temperature state, a cooling heat load of the cryoablation needle only exists in the target area 25 and tumor tissues outside the target area. Therefore, after switching to the freezing mode, the temperature of the distal end of the J-T slot can still be kept at the lowest temperature, and the outer wall of the target area 25 will be reduced from normal temperature to below -100°C instantly. In this way, surgical time for ablating a tumor with the same size is shortened, or a larger ablation range (ice ball) is produced within the same time. In addition, due to more rapid cooling of the tumor tissues, the probability of intracellular ice damage of tumor cells is greatly increased, and then freezing damage of the tumor cells is more thorough and the ablation effect is better.

In a preferred embodiment, the cryoablation needle having the J-T slot sleeve further includes: a J-T slot sleeve adjusting apparatus, where the J-T slot sleeve adjusting apparatus is configured to enable the distal end of the J-T slot sleeve to be switched between at least two adjusting positions.

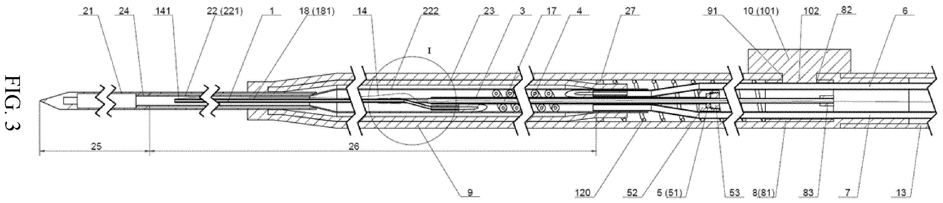

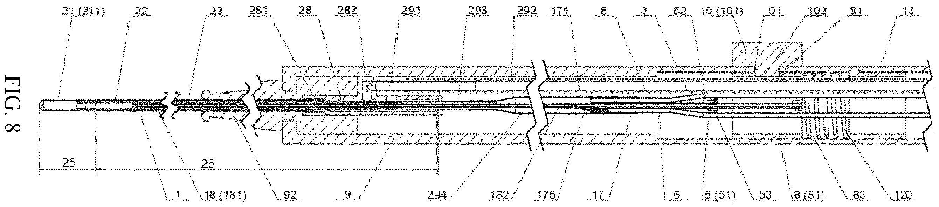

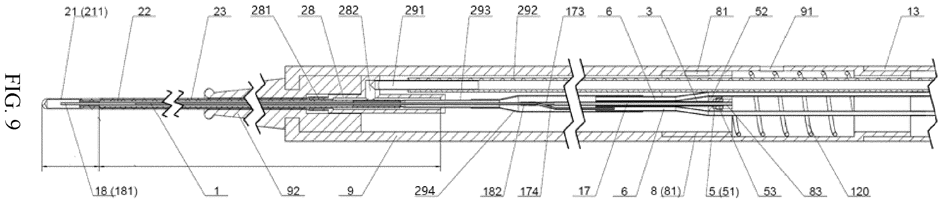

In an embodiment, the J-T slot sleeve adjusting apparatus includes: a push tube 17 and a mandrel 3. Refer to FIG. 3, FIG .5, FIG. 8 and FIG. 9. The mandrel 3 is arranged in the axis direction of the vacuum wall 2. The push tube 17 penetrates through the mandrel 3. A distal end of the push tube 17 is connected to a proximal end of the J-T slot sleeve 18. The distal end of the push tube 17 is an end of the push tube 17 close to the needle tip 211. The push tube 17 and the J-T slot sleeve 18 can be controlled to synchronously move in the axis direction, to switch the distal end of the J-T slot sleeve 18 between the adjusting positions.

In an embodiment, the cryoablation needle having the J-T slot sleeve further includes: a sealing assembly 5, where the sealing assembly 5 is configured to form a dynamic seal between the mandrel 3 and the push tube 17.

In an embodiment, the sealing assembly 5 includes: a sealing ring 51, a sealing slot 52 and a sealing press piece 53. Refer to FIG. 3, FIG .5, FIG. 8 and FIG. 9. The sealing ring 51 is placed in the sealing slot 52. The sealing press piece 53 is screwed into the sealing slot 52 in the axial direction, to fix the sealing ring 51 between the sealing slot 52 and the sealing press piece 53. The mandrel 3 is inserted into the sealing ring 51 and the sealing press piece 53, so that the sealing ring 51 is radially extruded and deformed between the mandrel 3 and the sealing slot 52 to form a dynamic seal. Optionally, the sealing ring 51 may be a rubber sealing ring, such as a Buna-N rubber O-shaped ring, or may be a low-temperature-resistant Variseal sealing ring including fluoropolymer and a metal spring.

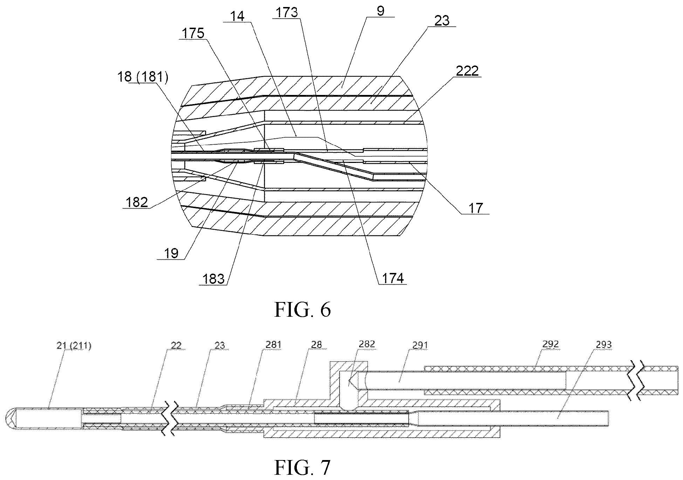

In a preferred embodiment, a vacuum wall of a flexible cryoablation needle may further includes: a vacuum tee 28, a vacuum connecting tube 291, a vacuum hose 292 and a return gas connecting tube 293. Refer to FIG. 7. A proximal end of the inner tube 22 is connected to and sealed with a distal end of the return gas connecting tube 293. A proximal end of the outer tube 23 is connected to and sealed with a tee connecting portion 281. A proximal end of the vacuum tee 28 is connected to and sealed with the return gas connecting tube 293. A distal end of the vacuum connecting tube 291 is inserted into a tee bypass 282. The vacuum hose 292 is inserted into the vacuum connecting tube 291. By vacuum-pumping a proximal end of the vacuum hose 292, a gap between the inner tube 22 and the outer tube 23 can be kept in a vacuum state, to prevent frostbite of a normal natural cavity wall.

In an embodiment, the cryoablation needle having the J-T slot sleeve further includes: a shunt 294, configured to seal gaps between a gas intake tube 6, a gas return tube 7 and the mandrel 3. The gas intake tube 6, the gas return tube 7 and the mandrel 3 are inserted into a proximal end of the shunt 294 for sealing. Refer to FG. 8 and FIG. 9.

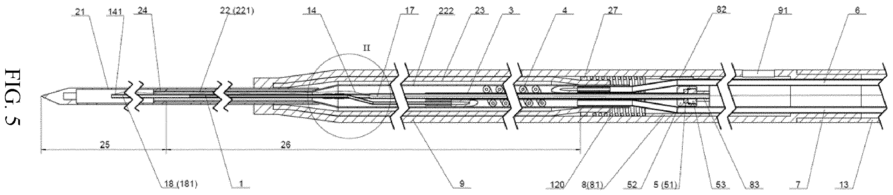

In an embodiment, position adjustment of the J-T slot sleeve can be achieved by manual forward and backward adjustment, or may be achieved by a prefabricated spring 120. The cryoablation needle having the J-T slot sleeve further includes: a clamping piece 10. One end of the spring 120 can move synchronously with the distal end of the push tube 17, and is also connected to the clamping piece 10. The clamping piece 10 can enter and exit from a clamped position. The other end of the spring 120 is fixed relative to the vacuum wall. When the clamping piece 10 is located at the clamped position, the spring 120 is limited by the clamping piece 10 to keep in a deformation state, and the distal end of the J-T slot sleeve 18 is located at the first adjusting position. Refer to FIG. 3 and FIG. 8. When the clamping piece 10 exits from the clamped position, the spring 120 can generate a restoring force for restoring from the deformation state to a natural state, and the restoring force can drive the push tube 17 to move, and then drive the J-T slot sleeve 18 to enter the second adjusting position from the first adjusting position. Refer to FIG. 5 and FIG. 9.

In an embodiment, the position of a distal end of the spring 120 is fixed relative to the vacuum wall. A proximal end of the spring 120 is connected to the clamping piece 10. When the distal end of the J-T slot sleeve 18 is located at the first adjusting position, the deformation state of the spring 120 is a tension state. Refer to FIG. 3. When the clamping piece 10 exits from the clamped position, the restoring force (tensile force) of the spring 120 drives the push tube to move toward the distal end, and then drives the J-T slot sleeve 18 to enter the second adjusting position from the first adjusting position. Refer to FIG 5.

In a different embodiment, it can also be set that the position of the proximal end of the spring 120 is fixed relative to the vacuum wall. The distal end of the spring 120 is connected to the clamping piece 10. When the clamping piece 10 is located at the clamped position, the spring 120 is limited by the clamping piece 10 to keep in a compression state, and the distal end of the J-T slot sleeve 18 is located at the first adjusting position. Refer to FIG. 8. When the clamping piece 10 exits from the clamped position, the restoring force (elastic force) of the spring 120 drives the push tube 17 to move toward the distal end, and then drives the J-T slot sleeve 18 to enter the second adjusting position from the first adjusting position. Refer to FIG. 9.

In an embodiment, the clamping piece 10 includes: a positioning pin 102, as shown in FIG. 3 and FIG. 8.

In an embodiment, the cryoablation needle having the J-T slot sleeve further includes: a sliding block 8 and a handle 9. Refer to FIG. 3, FIG .5, FIG. 8 and FIG. 9. The handle 9 is arranged at a proximal end of the cryoablation needle, and a position of the handle is fixed relative to the vacuum wall, which is convenient for grasping. The sliding block 8 is connected to a proximal end of the push tube 17, and the sliding block 8 and the clamping piece 10 are directly or indirectly connected. The sliding block 8, the push tube 17 and the J-T slot sleeve 18 can be controlled to synchronously move in the axis direction, to switch the distal end of the J-T slot sleeve 18 between the adjusting positions. The sliding block 8 includes: a guiding tube 81. The guiding tube 81 is arranged in the axis direction of the vacuum wall. The guiding tube 81 is provided with a middle fixing hole 83. The distal end of the push tube 17 is fixed to the middle fixing hole 83. The handle 9 is provided with a handle positioning slot 91. The sliding block 8 is provided with a sliding block positioning slot 82. When the distal end of the J-T slot is located at the first adjusting position, the positioning pin 102 is synchronously inserted into the handle positioning slot 91 and the sliding block positioning slot 82, so that the position of the sliding block 8 is fixed relative to the handle 9, that is, the current pre-purging mode is kept. When it needs to be switched to the freezing mode, only the positioning pin 102 needs to be pulled out of the handle positioning slot 91 and the sliding block positioning slot 82.

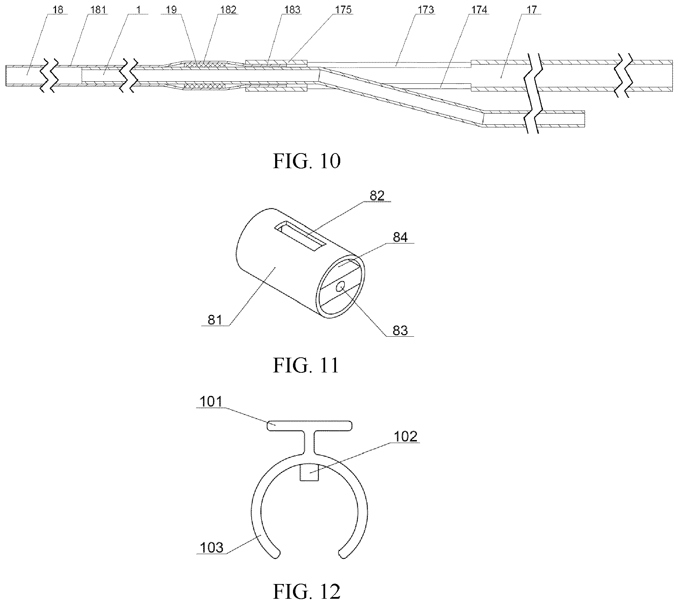

In an embodiment, the sliding block 8 further includes: a gas intake/return tube guiding hole 84 provided in the guiding tube 81. The gas intake tube 6 and the gas return tube 7 penetrate through the gas intake/return tube guiding hole 84. Refer to FIG. 11.

In a preferred embodiment, in order to facilitate the fixing of the clamping piece and the insertion and removal adjustment of the clamping piece, the clamping piece 10 further includes: a hand-held portion 101 and a C-shaped ring 103. Refer to FIG. 12. The hand-held portion 101 and the positioning pin 102 are arranged on the C-shaped ring 103. The C-shaped ring 103 is wrapped around the outer wall of the handle 9, which can prevent the clamping piece from falling off radially.

In a different embodiment, when the handle 9 is not included, the C-shaped ring 103 only needs to be wrapped around an outer wall with a fixed position relative to the vacuum wall, which can also achieve the purpose of preventing the clamping piece from falling off radially.

In a preferred embodiment, the cryoablation needle having the J-T slot sleeve further includes: a spring stop 27. The position of the spring stop 27 is fixed relative to the vacuum wall. Refer to FIG. 3 and FIG. 5, the distal end of the spring 120 is connected to the spring stop 27. The proximal end of the spring 120 is connected to the guiding tube 81 of the sliding block 8. The spring drives the sliding block, and then drives the push tube to move.

In a preferred embodiment, in order to improve a heat dissipation function, the cryoablation needle having the J-T slot sleeve further includes: a finned tube 4. The finned tube 4 is arranged on the outer wall of the mandrel 3. A proximal end of the finned tube 4 is hermetically connected to the gas intake tube 6, and a distal end of the finned tube 4 is hermetically connected to a proximal end of the J-T slot 1. Refer to FIG. 10, the proximal end of the J-T slot 1 is lead out from a gas intake notch 174, and then inserted into the distal end of the finned tube 4 for connection and sealing.

In an embodiment, the vacuum wall 2 further includes: an outer tube 23. Refer to FIG. 3, FIG. 6, FIG. 9 and FIG. 12. A distal end of the outer tube 23 is hermetically connected to a proximal end of the needle rod 21, and a proximal end of the outer tube 23 is hermetically connected to a proximal end of the inner tube 22.

In a preferred embodiment, in order to increase the internal volume of the proximal end of the inner tube, for example, the finned tube 4 may be inserted into the proximal end of the inner tube, or more other components can be accommodated. Since the internal volume of the proximal end of the inner tube needs to be increased, the internal volume of the proximal end of the vacuum wall also needs to be increased. An outer diameter of the outer tube 23 is greater than an outer diameter of the needle rod 21. An inner diameter of the outer tube 23 is greater than an inner diameter of the needle rod 21. The distal end of the outer tube 23 is an end of the outer tube 23 close to the needle tip 211. The proximal end of the outer tube 23 is an end of the outer tube 23 far away from the needle tip 211. Furthermore, from the distal end to the proximal end of the inner tube 22, the inner tube 22 sequentially includes: an inner tube front section 221 and an inner tube rear section 222. An outer diameter of the inner tube rear section 222 is greater than an outer diameter of the inner tube front section 221. An inner diameter of the inner tube rear section 222 is greater than an inner diameter of the inner tube front section 221. The inner tube front section 221 is located inside the needle rod 21. The inner tube rear section 222 is located inside the outer tube 23. A dynamic sealing point between the J-T slot sleeve and the J-T slot is arranged in the inner tube rear section 222. Refer to FIG. 3, FIG. 5, FIG. 8 and FIG. 9.

In an embodiment, the cryoablation needle having the J-T slot sleeve further includes: a gasket 24. Refer to FIG. 3 and FIG. 5. The gasket 24 is arranged between the outer wall of the distal end of the inner tube 22 and the inner wall of the needle rod 21 to form a sealed connection.

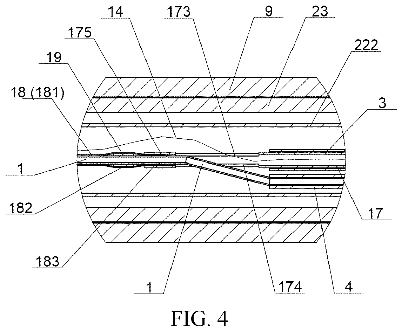

In an embodiment, the J-T slot sleeve 18 includes: a sleeve main section 181, a sleeve sealing section 182 and a sleeve connecting section 183. From the distal end to the proximal end of the J-T slot, the sleeve main section 181, the sleeve sealing section 182 and the sleeve connecting section 183 are distributed in sequence. Refer to FIG. 4 and FIG. 6. An inner diameter of the sleeve main section 181 is slightly greater than an outer diameter of the J-T slot 1. A sealing gasket 19 is placed between the sleeve sealing section 182 and the J-T slot 1. By extruding the sleeve sealing section 182 radially, a dynamic seal is formed for a gap between the J-T slot sleeve 18 and the J-T slot 1. The sealing gasket 19 is preferably made of PTFE materials. The sleeve sealing section 182 is preferably located inside the distal end of the inner tube rear section 222, to minimize its influence on return gas. An outer diameter of the sleeve connecting section 183 is also reduced by radial extrusion, and the sleeve connecting section 183 is inserted into and fixed to a push tube connecting section 175 of the push tube 17.

In a different embodiment, the dynamic seal between the J-T slot sleeve 18 and the J-T slot 1 can also be achieved by a Variseal sealing ring.

In a preferred embodiment, in order to better detect the freezing effect of the cryoablation needle, the cryoablation needle having the J-T slot sleeve further includes: a temperature measuring wire 14. A distal end of the temperature measuring wire 14 is a temperature measuring point 141, and the distal end of the temperature measuring wire 14 is an end of the temperature measuring wire 14 close to the needle tip 211. Refer to FIG. 3, FIG. 5, FIG. 8 and FIG. 9. The temperature measuring point 141 is arranged at the distal end of the J-T slot sleeve 18 and used for measuring temperature at the distal end of the J-T slot sleeve 18. When being located in the target area, the distal end of the J-T slot sleeve 18 is configured to monitor central temperature of a tumor during freezing and rewarming processes. When being located in the vacuum insulation area, the distal end of the J-T slot sleeve 18 is configured to indicate whether pre-purging is in place through the temperature during a pre-purging process. The temperature measuring wire 14 runs along the outer side of the J-T slot sleeve 18, and then leads out from the inside of the mandrel 3 or the push tube 17 to the outside, and glue is poured into the mandrel 3 or the push tube 17 for sealing. Referring to FIG. 10, the temperature measuring wire can be introduced into the inside of the push tube 17 through a temperature measuring notch 173, and glue is poured into the push tube (the section from a proximal end of the temperature measuring notch 173 to the proximal end of the push tube 17) for sealing. Preferably, the cryoablation needle having the J-T slot sleeve further includes a rewarming wire. The position and arrangement of the rewarming wire are consistent with those of the temperature measuring wire, and the rewarming wire is used for achieving a rewarming function. Preferably, the temperature measuring wire and/or the rewarming wire is a T-type enameled thermocouple wire.

In an embodiment, in order to wrap around components such as the gas intake tube and the gas return tube, and make the cryoablation needle cleaner in appearance and more convenient in operation, an outer sleeve 13 is further arranged on the outer wall of the handle 9. Refer to FIG. 3, FIG. 5, FIG. 8 and FIG. 9.

In a preferred embodiment, the pre-purging mode, that is, the distal end of the J-T slot sleeve being located in the vacuum insulation area 26, can be set to a factory delivery state of the product, and an operator can directly complete the pre-purging of the product through a needling test procedure. After needling test/pre-purging is completed, the product is further adjusted to the freezing mode, that is, the distal end of the J-T slot sleeve being located in the target area 25. After the freezing mode is enabled, since the cryoablation needle is pre-purged, the target area 25 will rapidly cool down to the lowest temperature.

In the description of this specification, description of reference terms such as "an implementation", "an embodiment" "a specific implementation process" or "an example" means including specific features, structures, materials, or characteristics described in the embodiment or example in at least one embodiment or example of the present invention. In this specification, exemplary descriptions of the foregoing terms do not necessarily refer to the same embodiment or example. Furthermore, specific features, structures, materials or characteristics described may be combined in any suitable manner in one or more embodiments or examples.

Finally, it should be noted that the foregoing embodiments are merely intended for describing the technical solutions of the present invention, but not for limiting the present invention. Although the present invention is described in detail with reference to the foregoing embodiments, persons of ordinary skill in the art should understand that they may still make modifications to the technical solutions described in the foregoing embodiments or make equivalent replacements to some or all technical features thereof, without making the essence of the corresponding technical solutions departing from the scope of the technical solutions of the embodiments of the present invention.

## Claims

1. A cryoablation needle having a J-T slot sleeve, comprising: a vacuum wall, a J-T slot and a J-T slot sleeve, wherein
the vacuum wall comprises: a needle rod and an inner tube;
the needle rod is provided with a needle tip at a distal end;
the inner tube penetrates through the needle rod, and a cavity is formed between the inner tube and the needle rod, the cavity being capable of forming a vacuum;
in an axis direction of the vacuum wall, a first preset distance exists between a distal end of the inner tube and the distal end of the needle rod; the distal end of the inner tube is an end of the inner tube close to the needle tip;
the J-T slot sleeve is sleeved outside a distal end of the J-T slot; the distal end of the J-T slot is an end of the J-T slot close to the needle tip;
the J-T slot and the J-T slot sleeve penetrate through the inner tube;
in areas in which the vacuum wall is distributed in the axis direction of the vacuum wall, an area in which the cavity is located is a vacuum insulation area, and an area in which the first preset distance exists is a target area;
the distal end of the J-T slot is located in the vacuum insulation area;
the J-T slot is capable of moving in the axis direction of the vacuum wall relative to the J-T slot, and a dynamic seal is formed between the J-T slot sleeve and the J-T slot;
a distal end of the J-T slot sleeve is capable of being switched between at least two adjusting positions relative to the vacuum wall, the at least two adjusting positions comprising: a first adjusting position and a second adjusting position;
the first adjusting position is located in the target area;
the second adjusting position is located in the vacuum insulation area;
when the distal end of the J-T slot sleeve is located at the first adjusting position, in the axis direction of the vacuum wall, a second preset distance exists between the distal end of the J-T slot sleeve and the needle tip, and the second preset distance at least ensures that an ice ball formed by freezing is wrapped around the needle tip; and
when the distal end of the J-T slot sleeve is located at the second adjusting position, in the axis direction of the vacuum wall, a third preset distance exists between the distal end of the J-T slot sleeve and a distal end of the vacuum insulation area, and the third preset distance at least ensures that a refrigerant directly returns from the inside of the vacuum insulation area after being sprayed from the J-T slot sleeve, the distal end of the vacuum insulation area being an end of the vacuum insulation area close to the needle tip.

2. The cryoablation needle having the J-T slot sleeve according to claim 1, further comprising: a J-T slot sleeve adjusting apparatus, wherein the J-T slot sleeve adjusting apparatus is configured to enable the distal end of the J-T slot sleeve to be switched between the at least two adjusting positions.

3. The cryoablation needle having the J-T slot sleeve according to claim 2, wherein the J-T slot sleeve adjusting apparatus comprises: a push tube and a mandrel, wherein
the mandrel is arranged in the axis direction of the vacuum wall;
the push tube penetrates through the mandrel;
a distal end of the push tube is connected to a proximal end of the J-T slot sleeve; the distal end of the push tube is an end of the push tube close to the needle tip; and
the push tube and the J-T slot sleeve are capable of being controlled to synchronously move in the axis direction, to switch the distal end of the J-T slot sleeve between the adjusting positions.

4. The cryoablation needle having the J-T slot sleeve according to claim 3, further comprising: a sealing assembly, wherein the sealing assembly is configured to form a dynamic seal between the mandrel and the push tube.

5. The cryoablation needle having the J-T slot sleeve according to claim 4, wherein the sealing assembly comprises: a sealing ring, a sealing slot and a sealing press piece, wherein
the sealing slot is fixedly sealed with a proximal end of the mandrel;
the sealing ring is arranged between the mandrel and the sealing slot, and the sealing press piece is arranged between the sealing ring and the sealing slot; and
the sealing press piece is capable of being controlled to extrude axially, so that the sealing ring radially extrudes the mandrel, to form the dynamic seal between the mandrel and the push tube.

6. The cryoablation needle having the J-T slot sleeve according to claim 4, further comprising: a spring and a clamping piece, wherein
one end of the spring is capable of moving synchronously with the distal end of the J-T slot sleeve, and is further connected to the clamping piece; the clamping piece is capable of entering and exiting from a clamped position;
the other end of the spring is fixed relative to the vacuum wall;
when the clamping piece is located at the clamped position, the spring is limited by the clamping piece to keep in a deformation state, and the distal end of the J-T slot sleeve is located at the second adjusting position;
the deformation state is a compression state or a tension state; and
when the clamping piece exits from the clamped position, the spring is capable of generating a restoring force for restoring from the deformation state to a natural state, and the restoring force is capable of driving the distal end of the J-T slot sleeve to enter the first adjusting position from the second adjusting position.

7. The cryoablation needle having the J-T slot sleeve according to claim 6, wherein the clamping piece comprises: a positioning pin and a C-shaped ring, wherein
the positioning pin is arranged on the C-shaped ring;
the C-shaped ring is wrapped around an outer wall with a fixed position relative to the vacuum wall; and
when the distal end of the J-T slot sleeve is located at the first adjusting position, the positioning pin is configured to keep the spring in the deformation state.

8. The cryoablation needle having the J-T slot sleeve according to claim 7, further comprising: a sliding block and a handle, wherein
the handle is arranged at a proximal end of the vacuum wall, and a position of the handle is fixed relative to the vacuum wall; the proximal end of the vacuum wall is an end of the vacuum wall far away from the needle tip;
the sliding block is connected to a proximal end of the push tube, and the sliding block is also directly or indirectly connected to the clamping piece; the proximal end of the push tube is an end of the push tube far away from the needle tip;
the sliding block, the push tube and the J-T slot sleeve are capable of being controlled to synchronously move in the axis direction, to switch the distal end of the J-T slot sleeve between the adjusting positions;
the handle is provided with a handle positioning slot and the sliding block is provided with a sliding block positioning slot; and
the positioning pin is capable of being controlled to be synchronously inserted into the sliding block positioning slot and the handle positioning slot, so that positions of the sliding block, the handle and the vacuum wall are relatively fixed, and in this case, the distal end of the J-T slot sleeve is located at the second adjusting position.

9. The cryoablation needle having the J-T slot sleeve according to claim 1, wherein the vacuum wall further comprises: an outer tube, wherein
a distal end of the outer tube is hermetically connected to a proximal end of the needle rod, a proximal end of the outer tube is hermetically connected to a proximal end of the inner tube; the distal end of the outer tube is an end of the outer tube close to the needle tip, and the proximal end of the outer tube is an end of the outer tube far away from the needle tip;
an outer diameter of the outer tube is greater than an outer diameter of the needle rod, and an inner diameter of the outer tube is greater than an inner diameter of the needle rod;
from the distal end to the proximal end of the inner tube, the inner tube sequentially comprises: an inner tube front section and an inner tube rear section; an outer diameter of the inner tube rear section is greater than an outer diameter of the inner tube front section; an inner diameter of the inner tube rear section is greater than an inner diameter of the inner tube front section; and
the inner tube front section penetrates through the needle rod; and the inner tube rear section penetrates through the outer tube.

10. The cryoablation needle having the J-T slot sleeve according to claim 9, wherein a dynamic sealing point between the J-T slot sleeve and the J-T slot is located at a proximal end of the J-T slot sleeve; and
the dynamic sealing point is located inside the inner tube rear section.

11. The cryoablation needle having the J-T slot sleeve according to any one of claims 1-10, further comprising: a temperature measuring wire, wherein
a distal end of the temperature measuring wire is a temperature measuring point; the distal end of the temperature measuring wire is an end of the temperature measuring wire close to the needle tip; and
the temperature measuring point is arranged at the distal end of the J-T slot sleeve and used for measuring temperature at the distal end of the J-T slot sleeve.
